# EUROPEAN PATENT SPECIFICATION

(11) **EP 3 040 421 B1**
(45) Date of publication and mention of the grant of the patent: **28.03.2018**
(21) Application number: 14839972.8
(22) Date of filing: 01.09.2014
(51) Int. Cl.: C12Q 1/60, C12Q 1/26, C12Q 1/32, C12Q 1/44, G01N 33/92

(54) **METHOD FOR MEASURING CHOLESTEROL IN HIGH-DENSITY LIPOPROTEIN, AND REAGENT FOR USE IN SAID METHOD**
VERFAHREN ZUR CHOLESTERINMESSUNG IN LIPOPROTEINEN VON HOHER DICHTE UND REAGENZ ZUR VERWENDUNG IN DIESEM VERFAHREN
PROCÉDÉ POUR MESURER LE CHOLESTÉROL DANS UNE LIPOPROTÉINE HAUTE DENSITÉ ET RÉACTIF DESTINÉ À ÊTRE UTILISÉ DANS LEDIT PROCÉDÉ

(30) Priority: 30.08.2013 JP 2013180140; 29.11.2013 JP 2013247966
(43) Date of publication of application: 06.07.2016
(73) Proprietor: Sekisui Medical Co., Ltd., Tokyo 103-0027 (JP)
(72) Inventor: OTA, Mieko, Tokyo 103-0027 (JP); OONO, Aiko, Tokyo 103-0027 (JP)
(74) Representative: Blodig, Wolfgang
(86) International application number: PCT/JP2014/072934
(87) International publication number: WO 2015/030236

(56) References cited:
- EP-A1- 1 876 243
- WO-A1-95/24502
- CA-A1- 2 828 934
- JP-A- H0 310 696
- JP-A- 2000 116 400
- JP-A- 2000 116 400
- US-A- 5 384 248
- US-A1- 2006 211 096
- US-A1- 2013 115 646
- BURSTEIN, M. ET AL.: 'Rapid method for the isolation of lipoproteins from human serum by precipitation with polyanions' JOURNAL OF LIPID RESEARCH vol. 11, no. 6, 1970, pages 583 - 595, XP000917101

## Description

### Field of the Invention

The present invention relates to a method for readily and correctly measuring cholesterol in high-density lipoproteins, and a reagent for use in the method.

### Background of the Invention

Cholesterol, neutral fat (hereinafter, also referred to as triglycerides or TGs) and phospholipids, which are the main lipids in the living body, exist together with apoproteins in blood to form lipoproteins. The lipoproteins are classified into such as chylomicrons, very low-density lipoproteins (hereinafter, also referred to as VLDL), low-density lipoproteins (hereinafter, also referred to as LDL), and high-density lipoproteins (hereinafter, also referredto as HDL) on the basis of the difference of physical property. Of these, HDL involves in removal action of cholesterol accumulated in cells, because HDL receives the cholesterol from each tissue in the living body. Therefore, HDL is known to be a risk prevention factor against various kinds of arteriosclerosis including coronary arteriosclerosis. Accordingly, obtaining the blood concentration of cholesterol in HDL (hereinafter, also referred to as HDL-C) is so useful for predicting the onsets of arteriosclerosis-related disease that the blood concentration thereof is widely measured in clinical sites.

Many kinds of clinical test reagent, which does not require a step of the separation and fractionation of HDL from lipoproteins other than HDL and whose use is classified into a so-called homogeneous HDL-C direct measuring method (hereinafter, also referred to as a conventional method), are commercially available and used in clinical test routinely. (Non-Patent documents 1 and 4). However, even such a practical method or clinical test reagent still has a problem in the measurement accuracy. In particular, in a specimen having an abnormal lipid condition, the difference between measured values obtained in accordance with reference methods (Non Patent Documents 2 and 3) and measured values obtained with various kinds of reagents was acknowledged as a problem internationally (Non Patent Document 4).

Most of the conventional methods can be classified as follows: a method to remove all or part of cholesterols in lipoproteins other than HDL from a reaction liquid prior to HDL-C measurement so that all or part of cholesterols in lipoproteins other than HDL cannot involved in the main reaction in the HDL-C measurement (hereinafter, also referred to as a main reaction simply) (for example, Patent Documents 1 and 2), or a method to inhibit reaction of cholesterols in lipoproteins other than HDL at the time of the HDL-C measurement so that the cholesterols in lipoproteins other than HDL cannot be involved in the main reaction in the HDL-C measurement (for example, Patent Documents 3 to 6). However, in such conventional methods, there has been a problem associated with elimination of the measurement error caused via VLDL, because the properties of VLDL and LDL, those are regarded as lipoproteins other than HDL, are not the same as each other.

### Patent Document

Patent Document 1: WO 98/26090 A
Patent Document 2: WO 2000/078999 A
Patent Document 3: WO 95/24502 A
Patent Document 4: WO 2004/035816 A
Patent Document 5: WO 2005/100591 A
Patent Document 6: WO 2006/118199 A

### Non Patent Document

Non Patent Document 1: Clinical Chemistry 47:9 1579-1596 (2001)
Non Patent Document 2: European Journal of Clinical Chemistry and Clinical Biochemistry 29 269-275 (1991)
Non Patent Document 3: Clinical Chemistry 45:10 1803-1812 (1999)
Non Patent Document 4: Clinical Chemistry 56:6 977-986 (2010)

### Summary of the Invention

### Problem to be solved by the Invention

Accordingly, an object of the present invention is to find out a compound capable of enhancing the consistency with the measured value by a standard measuring method such as DCM (Designated Comparison Method), even in a case where a specimen from a patient with, for example, dyslipidemia exhibiting a special disease state is used as a measurement sample, and to provide a method for measuring HDL-C with the use of the compound.

### Means for solving the problem

As a result of intensive studies on the above mentioned problem, the inventors found that, in the measurement of HDL-C, mixing enzymes for measuring cholesterol such as cholesterol oxidase with a measurement sample in the presence of a phosphonium salt and a polyanion allows the reactivity of the enzymes for measuring cholesterol with VLDL to be greatly inhibited, so that HDL-C can be measured with higher accuracy.

That is, the present invention relates to the followings.
[1] A method for measuring high-density lipoproteins cholesterol,
   comprising the step of reacting a measurement sample, and cholesterol ester hydrolase and cholesterol oxidase, or cholesterol ester hydrolase and cholesterol dehydrogenase, in the presence of a compound represented by general formula (I) : 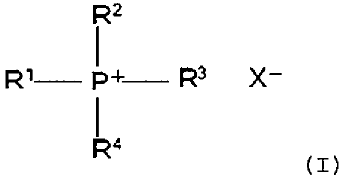
   wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion,
   and a polyanion.
[2] The method according to [1], wherein in the compound represented by general formula (I), R¹ represents a linear or branched alkyl group having 8 to 16 carbon atoms, and each of R², R³ and R⁴ is a linear or branched alkyl group having 1 to 4 carbon atoms.
[3] The method according to [1] or [2], wherein the compound represented by general formula (I) is at least one compound selected from the group consisting of trimethyloctylphosphonium salts, trimethyldodecylphosphonium salts,
   trimethylhexadecylphosphonium salts,
   triethyloctylphosphonium salts, triethyldodecylphosphonium salts, triethylhexadecylphosphonium salts,
   tributyloctylphosphonium salts, tributyldodecylphosphonium salts and tributylhexadecylphosphonium salts.
[4] The method according to any one of [1] to [3], wherein the polyanion is sodium phosphotungstate or dextran sulfate.
[5] The method according to any one of [1] to [4], wherein the above mentioned step is carried out further in the presence of albumin.
[6] The method according to any one of [1] to [5], wherein the above mentioned step is carried out further in the presence of at least one compound selected from the group consisting of polyoxyethylene alkylamines, polyoxyethylene alkenylamines, tertiary amines and quaternary ammonium salts.
[7] A reagent for measuring high-density lipoproteins cholesterol, comprising
   (a) a compound represented by general formula (I): 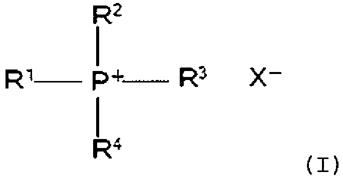 wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion,
   (b) a polyanion, and
   (c) cholesterol ester hydrolase and cholesterol oxidase, or cholesterol ester hydrolase and cholesterol dehydrogenase.
[8] The reagent according to [7], wherein in the compound represented by general formula (I), R¹ represents a linear or branched alkyl group having 8 to 16 carbon atoms, and each of R², R³ and R⁴ is a linear or branched alkyl group having 1 to 4 carbon atoms.
[9] The reagent according to [7] or [8], wherein the compound represented by general formula (I) is at least one compound selected from the group consisting of trimethyloctylphosphonium salts, trimethyldodecylphosphonium salts, trimethylhexadecylphosphonium salts, triethyloctylphosphonium salts, triethyldodecylphosphonium salts, triethylhexadecylphosphonium salts, tributyloctylphosphonium salts, tributyldodecylphosphonium salts and tributylhexadecylphosphonium salts.
[10] The reagent according to any one of [7] to [9], wherein the polyanion is from sodium phosphotungstate or dextran sulfate.
[11] The reagent described in any one of [7] to [10], further containing albumin.
[12] The reagent according to any one of [7] to [11], further containing at least one compound selected from the group consisting of polyoxyethylene alkylamines, polyoxyethylene alkenylamines, tertiary amines and quaternary ammonium salts.
[13] The reagent according to any one of [7] to [12], comprising
   a first reagent set containing (a) the compound represented by general formula (I) and (b) the polyanion, and
   a second reagent set containing (c) cholesterol ester hydrolase and cholesterol oxidase, or cholesterol ester hydrolase and cholesterol dehydrogenase.
[14] A kit for use in high-density lipoprotein cholesterol measurement, comprising
   (a) a compound represented by general formula (I): 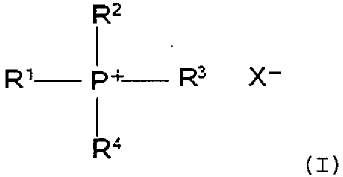 wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion,
   (b) a polyanion, and
   (c) cholesterol ester hydrolase and cholesterol oxidase, or cholesterol ester hydrolase and cholesterol dehydrogenase.
[15] The kit according to [14], wherein in the compound represented by general formula (I), R¹ represents a linear or branched alkyl group having 8 to 16 carbon atoms, and each of R², R³ and R⁴ is a linear or branched alkyl group having 1 to 4 carbon atoms.
[16] The kit according to [14] or [15], wherein the compound represented by general formula (I) is at least one compound selected from the group consisting of trimethyloctylphosphonium salts, trimethyldodecylphosphonium salts, trimethylhexadecylphosphonium salts, triethyloctylphosphonium salts, triethyldodecylphosphonium salts, triethylhexadecylphosphonium salts, tributyloctylphosphonium salts, tributyldodecylphosphonium salts and tributylhexadecylphosphonium salts.
[17] The kit according to any one of [14] to [16], wherein the polyanion is sodium phosphotungstate or dextran sulfate.
[18] The kit according to any one of [14] to [17], further containing albumin.
[19] The kit according to any one of [14] to [18], further containing at least one compound selected from the group consisting of polyoxyethylene alkylamines, polyoxyethylene alkenylamines, tertiary amines and quaternary ammonium salts.
[20] The kit according to any one of [14] to [19], comprising
   a first reagent set containing (a) the compound represented by general formula (I) and (b) the polyanion, and
   a second reagent set containing (c) cholesterol ester hydrolase and cholesterol oxidase, or cholesterol ester hydrolase and cholesterol dehydrogenase.

### Effects of the Invention

The present invention does not require pretreatment such as centrifugation of a measurement sample, and it enables to quantify HDL-C efficiently with a simple and easy operation. The present invention is also applicable to a variety of automatic analyzers and is extremely useful in a field of clinical examination as well, since it allows the carrying out of a specific measurement with a small amount of measurement sample. Moreover, the present invention has an advantage in that no load is applied on an analyzer, since in the HDL-C measurement of the present invention, the composition of the reagent for use in the measurement does not necessarily contain bivalent metal. Furthermore, the present invention has an advantage in that the measurement accuracy is greatly improved, in particular in a VLDL-rich specimen, in which a measurement error was caused by a conventional method. An example of the VLDL-rich specimen is a specimen such as IV-type hyperlipidemia, in which triglycerides are at a high value, whereas total cholesterol and LDL cholesterol are at an almost normal value.

### Description of Preferred Embodiments for carrying out the invention

In one embodiment, the present invention provides a method for measuring high-density lipoprotein cholesterol (HDL-C). The method of the present invention is carried out for a measurement sample containing HDL with the measurement of HDL-C with a publicly known reagent for measuring cholesterol in the presence of a polyanion and a phosphonium salt compound.

As the measurement sample for use in the method of the present invention, any specimen may be used as long as the specimen has a possibility of containing lipoproteins such as HDL, LDL, VLDL and chylomicrons . Examples thereof may include a body fluid such as serum and plasma or a dilution thereof, but are not limited thereto.

In the description, the enzyme for use in HDL-C measurement which utilizes cholesterol as a substrate is generally referred to as enzymes for measuring cholesterol. Examples of the enzymes for measuring cholesterol include cholesterol ester hydrolase (including the one classified into a lipase), cholesterol oxidase and cholesterol dehydrogenase.

Hereinafter, a description is made of the method of the present invention, using a two-steps (two reagents) HDL-C measuring system as a model, which is used frequently in a field of clinical test. The two-step measuring system (or two-reagent measuring system) is a method comprising carrying out a step (the first step) of inhibiting the reactivity of cholesterols in lipoproteins other than HDL with the enzymes for measuring cholesterol by using the first reagent at the beginning, and subsequently carrying out a step (the second step) of measuring cholesterol by adding the second reagent containing the enzymes for measuring cholesterol. In the following description, the first step and the second step in the two-step measuring system may be sometimes called simply the first step and the second step, respectively.

In the first step in the method of the present invention, a polyanion and a phosphonium salt compound are added to a measurement sample, and lipoproteins other than HDL in the measurement sample are treated with the polyanion and the phosphonium salt compound. Herein, the "treatment" means an electrostatically binding of polyanion and phosphonium salt compound to the surface of particles of lipoproteins other than HDL. In this step, depending on the added amount of the polyanion and the phosphonium salt compound, particles of lipoproteins other than HDL may gather (so-called aggregate) via the polyanion, but this cohesion phenomenon is not necessary to obtain the effect of the present invention. The first step is aimed at reducing the reactivity of cholesterols in lipoproteins other than HDL with the enzymes for measuring cholesterol in the following second step through the above mentioned "treatment".

Next, in the second step, HDL-C measurement is carried out with the use of a conventionally and publicly known method. Cholesterols contained in lipoproteins include esterified cholesterol (hereinafter, also referred to as cholesterol ester) and free cholesterol. Esterified cholesterol is hydrolyzed by cholesterol ester hydrolase so as to be converted into a free cholesterol and a fatty acid. The free cholesterol produced here becomes, together with originally existing free cholesterol, a substrate for the enzymes for measuring cholesterol (for example, cholesterol oxidase or cholesterol dehydrogenase). When cholesterol oxidase is used as the enzyme for measuring cholesterol, a free cholesterol is oxidized so as to be converted into a cholestenone and a hydrogen peroxide. Reacting to them a compound forming a quinone pigment by a reaction with hydrogen peroxide in the presence of a peroxidase, and measuring the absorbance of the produced quinone pigment allow the sum of esterified cholesterol and free cholesterol contained in HDL, that is the HDL-C amount to be measured. In addition, when cholesterol dehydrogenase is used as the enzyme for measuring cholesterol, measuring the increase of NAD (P) H that is a coenzyme on the basis of the absorbance allows the HDL-C amount to be measured.

The phosphonium salt compound used in the present invention includes a compound represented by general formula (I) (hereinafter, also referred to as compound (I)): 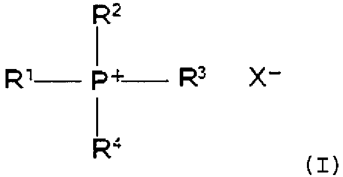 wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion.

Examples of the linear or branched alkyl group having 8 to 22 carbon atoms represented by R¹ in compound (I) include groups such as octyl, isooctyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, icosyl, henicosyl and docosyl (behenyl), in which an alkyl group having 8 to 18 carbon atoms is preferable, and a linear alkyl group having 12 to 16 carbon atoms is more preferable.

Examples of the linear or branched alkenyl group having 8 to 22 carbon atoms represented by R¹ in compound (I) include groups such as octenyl, nonenyl, decenyl, citronellyl, undecenyl, dodecenyl, tridecenyl, tetradecenyl, pentadecenyl, hexadecenyl, heptadecenyl, oleyl, nonadecenyl, icosenyl, henicosenyl and docosenyl, in which a linear alkenyl group having 8 to 18 carbon atoms is more preferable.

Examples of the linear or branched alkyl group having 1 to 6 carbon atoms represented by R², R³ or R⁴ in compound (I) include groups such as methyl, ethyl, propyl, butyl, pentyl and hexyl, in which a linear alkyl group having 1 to 4 carbon atoms is preferable.

Examples of the linear or branched alkenyl group having 2 to 6 carbon atoms represented by R², R³ or R⁴ in compound (I) include groups such as vinyl, 1-propenyl, allyl (2-propenyl), butenyl, pentenyl and hexenyl, in which a linear alkenyl group having 2 to 4 carbon atoms is more preferable.

Examples of the anion represented by X⁻ in compound (I) include a hydroxide ion, a halogen ion, an anion derived from an inorganic acid and an anion derived from an organic acid. Examples of the halogen ion include a fluorine ion, a chlorine ion, a bromine ion and an iodine ion. Examples of the anion derived from an inorganic acid include a nitrate ion, a sulfate ion, a phosphate ion, a carbonate ion and a tetrafluoroborate ion. Examples of the anion derived from an organic acid include a carboxylate ion such as a formate ion, an acetate ion, a lactate ion, a citrate ion and a glutaminate ion.

In the method of the present invention, as the phosphonium salt compound, above mentioned compound (I) may be used alone or in combination of two or more kinds thereof. Preferable examples of compound (I) include trimethyloctylphosphonium salts, trimethyldodecylphosphonium salts, trimethylhexadecylphosphonium salts, triethyloctylphosphonium salts, triethyldodecylphosphonium salts, triethylhexadecylphosphonium salts, tributyloctylphosphonium salts, tributyldodecylphosphonium salts, tributylhexadecylphosphonium salts and trihexyltetradecylphosphonium salts. The phosphonium salt preferably includes a salt with chloride and bromide . Specific examples (products) of compound (I) include tributyl-n-octyl phosphonium chloride, tributyldodecylphosphonium bromide, tributylhexadecylphosphonium bromide (which are all from Tokyo Chemical Industry Co., Ltd.), trihexyltetradecylphosphonium bromide, or trihexyltetradecylphosphoniumtetrafluoroborate (all from Sigma-Aldrich). In addition, with reference to descriptions of Green chemistry 5 143-152 (2003), Electrochemistry communications 9 2353-2358 (2007) and Electrochemistry 75 734-736 (2007), a person skilled in the art may also synthesize the phosphonium salt compound represented by formula (I), for example, trimethylhexadecylphosphonium bromide, triethyldodecylphosphonium bromide or triethylhexadecylphosphonium chloride.

The concentration of compound (I) is not limited in particular, as long as the concentration allows HDL-C measurement in the present invention. It is preferable that the concentration in a reaction liquid in the first step be from 0.001 mmol/L to 1 mol/L. It is more preferable that the concentration in the reaction liquid in the first step be from 0.01 to 10 mmol/L.

The polyanion is not limited in particular, as long as the polyanions allow HDL-C measurement in the present invention. Examples thereof include phosphotungstic acid or a salt thereof, dextran sulfate or a salt thereof, heparin or a salt thereof and polyethyleneglycol, but are not limited thereto. Examples of the dextran sulfate include dextran sulfates having an average molecular weight of from 4,000 to 2,000,000, preferably from 4,000 to 500,000, more preferably from 4,000 to 50,000, for example an average molecular weight of 4,000, 5,000, 10,000, 36,000, 40,000, 50,000, 80,000, 200,000, 500,000, 1,000,000 and 2, 000, 000. Examples of the salt thereof include a sodium salt, a potassium salt, a lithium salt, an ammonium salt and a magnesium salt. In addition, in the present invention, the above mentioned polyanion may be used alone or in combination of two or more kinds thereof . The concentration of the polyanion is not limited in particular, as long as the concentration allows HDL-C measurement in the present invention. It is preferable that the concentration in the reaction liquid in the first step be from 0.001 to 10% by weight. It is more preferable that the concentration in the reaction liquid in the first step be from 0.01 to 1% by weight.

Any enzyme for measuring cholesterol (for example, cholesterol ester hydrolase (including the one classified into lipase), cholesterol oxidase, and cholesterol dehydrogenase) and peroxidase may be used with no problem, as long as they can be used for measuring HDL-C. These may be derived from microorganism, animal or plant, or produced through gene manipulation, regardless of chemical modification. The enzymes for measuring cholesterol may be used alone or in combination of two or more kinds thereof . In addition, the used amount varies depending on the enzyme, but is not limited in particular. The enzyme may be used at a concentration of from 0.001 to 100 U/mL, preferably from 0.1 to 100 U/mL in a reaction liquid in the second step. The used amount of the peroxidase is also not limited, but the concentration is at from 0.001 to 100 U/mL, preferably at from 0.1 to 100 U/mL in the reaction liquid in the second step.

The compound forming a quinone pigment (hereinafter, also referred to as a color former) in reaction to hydrogen peroxide produced by the action of cholesterol oxidase in the presence of a peroxidase includes 4-aminoantipyrine and a Trinder's reagent. As the Trinder's reagent, N,N-bis(sulfobutyl)-m-toluidine-disodium (DSBmT), N-ethyl-N-(2-hydroxy-3-sulfopropyl)-3-methylaniline (TOOS), N-ethyl-N-(3-methylphenyl)-N'-succinylethylenediamine (EMSE), and the like are used for example, but the Trinder's reagent is not limited thereto. In addition, the concentration of the color former used may be selected appropriately, depending on the formed amount of a quinone pigment, the amount of a reaction liquid and the reaction time that are desired, for example.

In addition, when cholesterol dehydrogenase is used, the concentration of the coenzyme NAD (P) used may also be selected appropriately.

In order to advantageously carry out the method of the present invention, a reagent may be used which is prepared by appropriately combining, for example, the above mentioned polyanion, phosphonium salt compound, enzyme, color former and coenzyme. In addition, if necessary, an enzyme other than the enzymes for measuring cholesterol, such as an ascorbic acid oxidase, a catalase, a phospholipase or a sphingomyelinase, a salt, a buffer for pH adjustment, a surfactant, a protein such as albumin, an antibody, an antibiotic, a substance having an affinity to a specific lipoprotein, such as saponin, lectin, digitonin, cyclodextrin or calixarene, a stabilizer such as a chelator or sugar, or a preservative maybe further incorporated. In addition, since bivalent metals such as magnesium ions or calcium ions may contribute to the stabilization of the enzyme, they may be added at a concentration so as not to affect HDL-C measurement (or to affect a measuring device).

Of these, any buffer, examples thereof including a Good's buffer, phosphoric acid, Tris or phthalate, may be used, as long as the buffer may serve as a reaction liquid capable of setting a condition of having a buffer action in a pH range from 4 to 10. The used amount is not limited in particular, but the buffer is used at from 0.0005 to 2 mol/L, preferably at from 0.01 to 1 mol/L in a reaction liquid. In any embodiments, the optimum condition may be experimentally selected, on the basis of for example the characteristic of a variety of enzymes for use, or the relationship with another component included in the reagent.

Albumin may improve the sensitivity and the specificity in the present invention, and so albumin may be suitably used. The kind is not limited in particular, as long as the albumin allows HDL-C measurement in the present invention. Examples thereof include albumins derived from cattle, horse, sheep, human andeggwhite, in which a bovine serum albumin (BSA) is preferable. These may also be produced through gene manipulation, regardless of purification method, and regardless of chemical modification. The used amount is not limited in particular, but the concentration is preferably at from 0.001 to 10% by weight, more preferably at from 0.1 to 1% by weight in a reaction liquid.

The surfactant is not limited in particular, as long as the surfactant allows HDL-C measurement in the present invention, and thus may be appropriately selected for use from conventionally and publicly known surfactants (a non-ionic surfactant, an anionic surfactant, a cationic surfactant and an amphoteric surfactant) for use in HDL-C measurement.

For the purpose of selectively solubilizing HDL in particular, polyoxyethylene derivatives of HLB 13 to 14 may be suitably used, in which polyoxyethylene tribenzyl phenyl ether is more preferable. Specific examples (product names) include EMULGEN A-90 and EMULGEN B-66 (both from Kao Corporation) . The concentration is preferably at from 0.05 to 3% by weight, more preferably at from 0.1 to 1. 5% by weight in the reaction liquid in the second step (expressed in another way, with respect to the whole reaction mixture in the first step, the second step) . In addition, polycyclic polyoxyalkylene derivative described in WO 2004/048605 A may also be used in the same way.

In addition, as the surfactant, polyoxyethylene alkylamine or polyoxyethylene alkenylamine may be added. Examples of the polyoxyethylene alkylamine or the polyoxyethylene alkenylamine include compounds represented by general formula (II) (hereinafter, also referred to as compound (II)) : 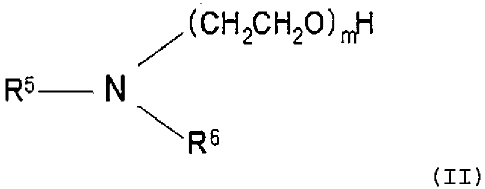 wherein R⁵ represents a linear or branched alkyl group or alkenyl group, R⁶ represents a hydrogen atom or (CH₂CH₂O)ₙH, and m and n are the same as or different from each other and are each an integer from 1 to 100. Examples of the alkyl group and alkenyl group in compound (II) include the linear or branched alkyl group having 8 to 22 carbon atoms and the linear or branched alkenyl group having 8 to 22 carbon atoms as mentioned above in compound (I), respectively, in which the alkyl group or alkenyl group having 10 to 18 carbon atoms is preferable.

Specific examples (products) of the polyoxyethylene alkylamine or the polyoxyethylene alkenylamine include NYMEEN L-201 (N-hydroxyethyl-laurylamine; NOF CORPORATION), NYMEEN L-207 (polyoxyethylene laurylamine; NOF CORPORATION), NYMEEN S-204, NYMEEN S-210 (polyoxyethylene stearylamine; NOF CORPORATION), Newcol OD-420 (a polyoxyethylene alkylamine ether; Nippon Nyukazai Co., Ltd.), Pionin D-3104, PioninD-3110 (polyoxyethylene lauryl amino ether; TAKEMOTO OIL FAT Co., Ltd.), BLAUNON L-205, L-207, L-210 (polyoxyethylene laurylamine; AOKI OIL INDUSTRIAL Co. , Ltd.), BLAUNONS-207, S-210 (polyoxyethylene stearylamine; AOKI OIL INDUSTRIAL Co., Ltd.), BLAUNON S-207T (a polyoxyethylene beef tallow amine; AOKI OIL INDUSTRIAL Co. , Ltd.) and BLAUNON O-205 (polyoxyethylene oleylamine; AOKI OIL INDUSTRIAL Co., Ltd.).

The degree of polymerization of the oxyethylene chain in each of the polyoxyethylene alkylamine and the polyoxyethylene alkenylamine is preferably from 1 to 100, more preferably from 1 to 60. The polyoxyethylene alkylamine and the polyoxyethylene alkenylamine may be used alone or in combination of two or more kinds thereof. In addition, the used amount is not limited in particular, but the concentration is preferably at from 0.0001 to 1% by weight, more preferably at from 0.001 to 0.1% by weight in a reaction liquid.

In addition, as the surfactant, a cationic surfactant or a surfactant having an amino group may be included. Examples thereof include a tertiary amine and a quaternary ammonium salt. Examples of the tertiary amine include compounds represented by general formula (III) (hereinafter, also referred to as compound (III)): 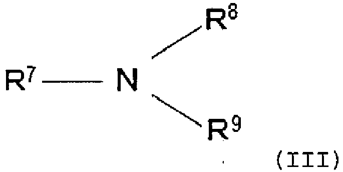 wherein R⁷ represents a linear or branched alkyl group or alkenyl group having 6 to 30 carbon atoms, and R⁸ and R⁹ are the same as or different from each other and each represent a hydrogen atom, a linear or branched alkyl group having 1 to 30 carbon atoms or a linear or branched alkenyl group having 2 to 30 carbon atoms. The specific examples (products) of compound (III) include NISSAN AMINE BB, NISSAN AMINE AB, TERTIARY NISSAN AMINE BB, TERTIARY NISSAN AMINE FB (which are all fromNOF CORPORATION) . The used amount of the tertiary amine and the quaternary ammonium salt is not limited in particular, but the concentration is preferably at from 0.0001 to 1% by weight, more preferably at from 0.001 to 0.1% by weight in a reaction liquid. The above mentioned tertiary amine and quaternary ammonium may be used alone or in combination of two or more kinds thereof.

In order to advantageously carry out the method of the present invention, a kit collectively including the above mentioned reagents for use in the method of the present invention may also be provided. Accordingly, in another embodiment, the present invention provides a reagent for measuring HDL-C, including the above mentioned phosphonium salt compound represented by compound (I), the above mentioned polyanion and the above mentioned enzymes for measuring cholesterol. In yet another embodiment, the present invention provides a kit for use in HDL-C measurement, containing the above mentioned phosphonium salt compound represented by compound (I), the above mentioned polyanion and the above mentioned enzymes for measuring cholesterol. The reagent for measuring HDL-C and the kit may also contain another reagent which is required for HDL-C measurement, such as the above mentioned peroxidase, color former, coenzyme, protein such as albumin, surfactant, enzyme other than the enzymes for measuring cholesterol, salt, buffer for pH adjustment, antibody, antibiotic, substance having an affinity to a specific lipoprotein, chelator, stabilizer, preservative or bivalent metal contributing to the stabilization of the enzyme.

One specific example of the reagent for measuring HDL-C (for the two-step measuring system) of the present invention is as follows.

### (First reagent)

### Polyanion

Phosphonium salt compound (compound (I))
Color former (for example, 4-aminoantipyrine or Trinder's reagent)

### (Second reagent)

Enzymes for measuring cholesterol
Cholesterol ester hydrolase
Cholesterol oxidase

### Peroxidase

Color former (for example, 4-aminoantipyrine or Trinder's reagent)

### Surfactant

Another specific example of the reagent for measuring HDL-C (for the two-step measuring system) of the present invention is as follows.

### (First reagent)

### Polyanion

Phosphonium salt compound (compound (I))
Coenzyme (NAD(P))

### (Second reagent)

Enzymes for measuring cholesterol
Cholesterol ester hydrolase
Cholesterol dehydrogenase

### Coenzyme (NAD(P))

### Surfactant

The description on the basis of the above mentioned two-step measuring system is made for the purpose of promoting understanding of the method of the present invention, but not for the purpose of binding the method of the present invention to a specific theory. Accordingly, the above mentioned description does not aim at excluding the possibility that the method of the present invention is a one-step (one-reagent) measuring system or a three steps (three reagents) measuring system, to the extent that the treatment of the present invention is substantially carried out before or in parallel to the contact of the enzymes for measuring cholesterol with lipoproteins. Furthermore, it also does not aim at excluding the possibility that the method of the present invention is a system other than the liquid reacting system where the reaction is carried out in a solution (for example, a solid reacting system where for example a test piece to which a reagent is fixed, or a dry analysis element is used). It goes without saying that, taking into consideration the detailed description of the method of the present invention on the basis of the above mentioned two-step measuring system or the description of the following Examples, a person skilled in the art could apply the method of the present invention to a one-step measuring system or a three-step measuring system, or further to a reacting system where a test piece or a dry analysis element is used, and experimentally determine the proper amount of each reagent used in the measuring system and the reacting system.

For example, in the reagent for measuring HDL-C as exemplified above, to the extent that the performance is not lost, it is possible to select whether for example the above mentioned enzymes such as the enzymes for measuring cholesterol and peroxidase, the color former and the coenzyme are included in the first reagent only, in the second reagent only, or in both reagents. In addition, with reference to WO 02/38800 A, JP 2007-325587 A, JP 2009-125049 A and JP 2009-247317 A and so on, the above mentioned reagent may be applied to for example a test piece or a dry analysis element. It goes without saying that a further component or technology usable for HDL-C measurement, on the basis of for example a test piece or a dry analysis element, may be used in the method of the present invention.

In the method of the present invention, because the involvement of cholesterols in lipoproteins other than HDL in the main reaction is inhibited, the cholesterols remain in the reaction liquid even after HDL-C measurement. Accordingly, further performing a means for cancelling the reaction inhibition of cholesterols other than HDL-C after HDL-C measurement (for example, addition of a surfactant capable of solubilizing all lipoproteins) allows the cholesterols in lipoproteins other than HDL (so-calledanonHDL-C inclinical, for example) to be measured. In addition, in the method of the present invention, using enzymes for measuring triglycerides, which is well known to a person skilled in the art, instead of the enzymes for measuring cholesterol, also allows triglycerides in HDL or triglycerides in lipoproteins other than HDL to be measured.

### Examples

Next, a more detailed description is made of the present invention with reference to Examples, but the present invention is not limited thereto. In the Examples and Comparative Examples, reagents and enzymes from the following manufacturers were used. The component names of surfactants are described in accordance with the names listed in catalogs from the individual manufacturers.

MES (DOJINDO LABORATORIES), sodium hydroxide (Kishida Chemical Co., Ltd.), TOOS (DOJINDO LABORATORIES), 4-aminoantipyrine (Wako Pure Chemical Industries, Ltd.), sodium phosphotungstate (Kishida Chemical Co., Ltd.), sodium dextran sulfate (molecular weight of 4,000) (Tokyo Chemical Industry Co. , Ltd.), sodium dextran sulfate (molecular weight of 36,000 to 50, 000) (Wako Pure Chemical Industries, Ltd.), sodium dextran sulfate (molecular weight of 500,000) (Wako Pure Chemical Industries, Ltd.), magnesium chloride (Kishida Chemical Co., Ltd.), tributyl-n-octyl phosphonium chloride (Tokyo Chemical Industry Co. , Ltd.), tributyl dodecyl phosphonium bromide (Tokyo Chemical Industry Co., Ltd.), tributyl hexadecyl phosphonium bromide (Tokyo Chemical Industry Co., Ltd.), trihexyl tetradecyl phosphonium bromide (Tokyo Chemical Industry Co., Ltd.), cholesterol esterase (Asahi Kasei Corporation), cholesterol oxidase (Oriental Yeast Co., Ltd.), peroxidase (TOYOBO Co. , Ltd.), EMULGEN B-66 (component name: polyoxyethylene tribenzyl phenyl ether; Kao Corporation), bovine serum albumin (BSA; Sigma), BLAUNON L-205 (component name: polyoxyethylene laurylamine; AOKI OIL INDUSTRIAL Co., Ltd.), BLAUNON S-207 (component name: polyoxyethylene stearylamine; AOKI OIL INDUSTRIAL Co., Ltd.), cetyltrimethylammonium bromide (Tokyo Chemical Industry Co., Ltd.), TERTIARY NISSAN AMINE BB (component name: dimethyl laurylamine; NOF CORPORATION), Pegnol 005 (component name: polyoxyethylene tribenzyl phenyl ether; TOHO CHEMICAL INDUSTRY Co., Ltd.), EMULGEN A-60 (component name: polyoxyethylene distyrenated phenyl ether; Kao Corporation), Newcol 610 (component name: polyoxyethylene polycyclic phenyl ether; Nippon Nyukazai Co., Ltd.), Newcol 2600FB (component name: polyoxyalkylene polycyclic phenyl ether; Nippon Nyukazai Co. , Ltd.), sodium cholate (Wako Pure Chemical Industries, Ltd.), ENAGICOL L-30AN (component name: alaninate; Lion Corporation), AMPHITOL 24B (component name: betaine lauryldimethylaminoacetate; Kao Corporation), ENAGICOL C-40H (component name: imidazolinium betaine; Lion Corporation). Trimethylhexadecylphosphonium bromide (CAS number [71221-96-0]), triethyldodecylphosphonium bromide (CAS number [21743-53-3]), triethylhexadecylphosphonium chloride (CAS number [56155-08-9]) were synthesized in accordance with a method described in Electrochemistry communications 9 2353-2358 (2007).

### (Comparative Example 1) Comparative evaluation of characteristics in accordance with conventional methods

Characteristics of conventional methods were evaluated by comparison, with samples of HDL fractions or VLDL fractions prepared through ultracentrifugation, which were diluted with a saline such that the concentration of cholesterol in each of the fractions became 50 mg/dL (hereinafter, the samples are also referred to as HDL fraction samples or VLDL fraction samples) . The evaluation was carried out, with a reagent (Comparative Example 1-1) which was prepared by adding only a polyanion to the first reagent (hereinafter, also referred to as a basic first reagent) represented as follows, a reagent (Comparative Example 1-2) which was prepared by adding to the first reagent a combination of compounds described in Patent Literature 3, a reagent (Comparative Example 1-3) which was prepared by adding to the first reagent compounds described in Patent Literature 4, and reagents (Comparative Example 1-4, Comparative Example 1-5) which were prepared by adding to the first reagent compounds described in Patent Literature 6. The names and concentrations of the components each added to the basic first reagent are indicated in Table 1. As a second reagent (hereinafter, also referred to as a basic second reagent), a reagent having the composition represented as follows was used.

**First reagent (basic first reagent)**

| | |
|---|---|
| MES-NaOH buffer solution (pH 6.5) | 100 mmol/L |
| 4-aminoantipyrine | 0.7 mmol/L |

**Second reagent (basic second reagent)**

| | |
|---|---|
| MES-NaOH buffer solution (pH 6.5) | 100 mmol/L |
| Cholesterol esterase | 1 U/mL |
| Cholesterol oxidase | 1 U/mL |
| Peroxidase | 5 U/mL |
| TOOS | 0.1 mmol/L |
| EMULGEN B-66 | 1.0% by weight |

**[Table 1]**

| NAME OF FIRST REAGENT | |
|---|---|
| COMPARATIVE EXAMPLE 1-1 | SODIUM PHOSPHOTUNGSTATE 0.04% BY WEIGHT |
| COMPARATIVE EXAMPLE 1-2 | SODIUM PHOSPHOTUNGSTATE 0.04% BY WEIGHT |
| | MAGNESIUM CHLORIDE 8 mmol/L |
| COMPARATIVE EXAMPLE 1-3 | SODIUMDEXTRAN SULFATE (MOLECULAR WEIGHT OF 500, 000) 0.1% BY WEIGHT |
| | BLAUNON L-205 0.007% BY WEIGHT |
| | BSA 0.2% BY WEIGHT |
| COMPARATIVE EXAMPLE 1-4 | SODIUMDEXTRAN SULFATE (MOLECULAR WEIGHT OF 500, 000) 0.1% BY WEIGHT |
| | CETYLTRIMETHYLAMMONIUM BROMIDE 0.014% BY WEIGHT |
| | BSA 0.2% BY WEIGHT |
| COMPARATIVE EXAMPLE 1-5 | SODIUM PHOSPHOTUNGSTATE 0.04% BY WEIGHT |
| | CETYLTRIMETHYLAMMONIUM BROMIDE 0.014% BY WEIGHT |
| | BSA 0.2% BY WEIGHT |

Measurement was carried out with Hitachi 7170 automatic analyzer (Hitachi High-Technologies Corporation). To 2.4 µL of each of the HDL fraction samples or the VLDL fraction samples, was added 240 µL of the first reagent of each of the Comparative Examples, and approximately five minutes later, 80 µL of the basic second reagent was further added. The absorbances just before the addition of the basic second reagent and five minutes after the addition thereof were measured, and the difference was determined between them (a two-point method). The measurement of absorbance was carried out at a main wavelength of 600 nm and a complementary wavelength of 700 nm.

The absorbance of the HDL fraction sample measured with the first reagent of Comparative Example 1-1 was indexed as 100, the relative absorbance (%) of the HDL fraction sample measured with each of the first reagents of Comparative Example 1-2 to Comparative Example 1-5 (the absorbance of the HDL fraction sample of each of the Comparative Examples × 100/the absorbance of the HDL fraction sample of Comparative Example 1-1) was determined. Furthermore, for the measurement with each of the first reagents, the relative absorbance (%) of the VLDL fraction sample with respect to the absorbance of the HDL fraction sample (the absorbance of the VLDL fraction sample × 100/the absorbance of the HDL fraction sample in the same Comparative Example) was calculated. A condition where the relative absorbance of the HDL fraction sample is large and the relative absorbance of the VLDL fraction sample/HDL fraction sample is small is required to achieve an object of the present invention; that is, a condition where the reactivity of the enzyme for measuring cholesterol with HDL-C is maintained, while the reactivity of the enzyme for measuring cholesterol with VLDL-C is lowered. The results are shown in Table 2.

**[Table 2]**

| NAME OF FIRST REAGENT | RELATIVE ABSORBANCE (%) OF HDL FRACTION SAMPLE | RELATIVE ABSORBANCE (%) OF VLDL FRACTION SAMPLE/HDL FRACTION SAMPLE |
|---|---|---|
| COMPARATIVE EXAMPLE 1-1 | 100 | 19.1 |
| COMPARATIVE EXAMPLE 1-2 | 104 | 7.3 |
| COMPARATIVE EXAMPLE 1-3 | 96.8 | 13.7 |
| COMPARATIVE EXAMPLE 1-4 | 27.7 | 14.0 |
| COMPARATIVE EXAMPLE 1-5 | 31.1 | 189.0 |

First of all, the relative absorbance (%) of the HDL fraction sample represents the relative strength of reactivity of each of the first reagents of the Comparative Examples with the HDL fraction. Accordingly, when the numerical value is smaller than 100, it is indicated that the reactivity with the HDL fraction decreases in comparison with Comparative Example 1-1. Next, the relative absorbance (%) of the VLDL fraction sample/the HDL fraction sample reflects the difference in reactivity with both of the fractions, because the concentration of cholesterol in the HDL fraction sample is the same as that in the VLDL fraction sample. When the numerical value is 100, it is indicated that the reactivity with the HDL fraction is equal to that with the VLDL fraction, and when the numerical value is smaller, it is indicated that the reactivity with the VLDL fraction is lower. The relative absorbance of the VLDL fraction sample with respect to the HDL fraction sample of Comparative Example 1-1 is 19.1%. It can be considered as one of the conditions that enable to inhibit the reactivity of the VLDL fraction in relation to the HDL fraction. Comparative Example 1-2 (Patent Literature 3) is of a test condition on the basis of the reagent inwhichabivalent metal salt further coexist with the basic first reagent. It enables to further decrease the reactivity with the VLDL fraction while maintaining the reactivity with the HDL fraction in relation to Comparative Example 1-1. However, when the bivalent metal salt (in particular, a magnesium salt) is mixed with an alkaline cleaning liquid, which is a passage cleaner for the automatic analyzer, a hardly water-soluble hydroxide salt (magnesium hydroxide) is formed to cause a serious problem such as clogging of passage, associated with maintenance and management of the apparatus. Such a problem is recognized as a problem of the conventional technology. In Comparative Example 1-3 (Patent Literature 4), the reactivity with the VLDL fraction slightly decreases in comparison with Comparative Example 1-1 in which a polyanion is used alone. However, the reactivity cannot be inhibited as much as done in Comparative Example 1-2 in which magnesium ions are used. In addition, it is found that in Comparative Examples 1-4 and 1-5 (Patent Literature 6), not only the reactivity with the VLDL fraction cannot be inhibited (inparticular, Comparative Example 1-5), but also the reactivity with the HDL fraction is lowered.

### (Example 1) Screening of a compound effective for solving the problem

With samples of HDL fractions or VLDL fractions prepared through ultracentrifugation, and which were those diluted with a saline such that the concentration of cholesterol in each of the fractions became 50 mg/dL (hereinafter, the samples are also referred to as HDL fraction samples or VLDL fraction samples), screening of a compound capable of lowering the reactivity of the enzymes for measuring cholesterol with the VLDL fraction was carried out. Reagents used for the screening were those (Example 1-1 to Examples 1-16) prepared by adding a polyanion, a variety of phosphonium salt compounds and a BSA to the basic first reagent described in Comparative Example 1, so as to reach the concentration indicated in Table 3. As the second reagent, the basic second reagent described in Comparative Example 1 was used.

**[Table 3]**

| NAME OF FIRST REAGENT | POLYANION | PHOSPHONIUM SALT COMPOUND | | BSA CONCENTRA TION % BY WEIGHT |
|---|---|---|---|---|
| | | NAME | CONCENTRA TION mmol/L | |
| COMPARATIVE EXAMPLE 1-1 | DS (MOLECULAR WEIGHT OF 4,000) | P₁₁₁₍₁₆₎ | 0.1 | 1.0 |
| COMPARATIVE EXAMPLE 1-2 | PTA | P₂₂₂₍₁₂₎ | 0.25 | 1.0 |
| COMPARATIVE EXAMPLE 1-3 | DS (MOLECULAR WEIGHT OF 4,000) | P₂₂₂₍₁₂₎ | 0.25 | 1.0 |
| COMPARATIVE EXAMPLE 1-4 | DS (MOLECULAR WEIGHT OF 36,000 TO 50,000) | P₂₂₂₍₁₂₎ | 0.25 | 1.0 |
| COMPARATIVE EXAMPLE 1-5 | DS (MOLECULAR WEIGHT OF 500,000) | P₂₂₂₍₁₂₎ | 0.25 | 1.0 |
| COMPARATIVE EXAMPLE 1-6 | DS (MOLECULAR WEIGHT OF 4,000) | P₂₂₂₍₁₆₎ | 0.1 | 1.0 |
| COMPARATIVE EXAMPLE 1-7 | PTA | P₄₄₄₈ | 0.4 | 1.0 |
| COMPARATIVE EXAMPLE 1-8 | PTA | P₄₄₄₈ | 1.5 | 1.0 |
| COMPARATIVE EXAMPLE 1-9 | PTA | P₄₄₄₍₁₂₎ | 0.05 | 0.0 |
| COMPARATIVE EXAMPLE 1-10 | PTA | P₄₄₄₍₁₂₎ | 0.05 | 1.0 |
| COMPARATIVE EXAMPLE 1-11 | DS (MOLECULAR WEIGHT OF 500,000) | P₄₄₄₍₁₂₎ | 0.005 | 1.0 |
| COMPARATIVE EXAMPLE 1-12 | DS (MOLECULAR WEIGHT OF 500,000) | P₄₄₄₍₁₂₎ | 0.01 | 1.0 |
| COMPARATIVE EXAMPLE 1-13 | PTA | P₄₄₄₍₁₆₎ | 0.01 | 1.0 |
| COMPARATIVE EXAMPLE 1-14 | PTA | P₄₄₄₍₁₆₎ | 0.07 | 1.0 |
| COMPARATIVE EXAMPLE 1-15 | PTA | P₆₆₆₍₁₄₎ | 0.05 | 1.0 |
| COMPARATIVE EXAMPLE 1-16 | PTA | P₆₆₆₍₁₄₎ | 0.2 | 1.0 |

In the Table, PTA represents sodium phosphotungstate, which was used at 0.04% by weight. DS represents sodium dextran sulfate, which was used at 0.05% by weight. In addition, as to the phosphonium salt compound, P₁₁₁₍₁₆₎ represents trimethylhexadecylphosphonium bromide, P₂₂₂₍₁₂₎ does triethyldodecylphosphonium bromide, P₂₂₂₍₁₆₎ does triethylhexadecylphosphonium chloride, P₄₄₄₈ does tributyl-n-octyl phosphonium chloride, P₄₄₄₍₁₂₎ does tributyldodecylphosphonium bromide, P₄₄₄₍₁₆₎ does tributylhexadecylphosphonium bromide, and P₆₆₆₍₁₄₎ does trihexyltetradecylphosphonium bromide. The concentration represents a concentration in the first reagent in any case.

Measurement and analysis of the results were carried out in the same way as Comparative Example 1. Namely, the absorbance of the HDL fraction sample measured with the first reagent of Comparative Example 1-1 was indexed as 100, and the relative absorbance (%) of the HDL fraction sample measured with the first reagent of each of the Examples was determined. Furthermore, for the measurement with each of the first reagents, the relative absorbance (%) of the VLDL fraction sample with respect to the absorbance of the HDL fraction sample was calculated. The results are shown in Table 4.

**[Table 4]**

| NAME OF FIRST RE AGENT | RELATIVE ABSORBANCE (%) OF HDL FRACTION SAMPLE | RELATIVE ABSORBANCE (%) OF VLDL FRACTION SAMPLE/HDL FRACTION SAMPLE |
|---|---|---|
| COMPARATIVE EXAMPLE 1-1 | 100.0 | 19.1 |
| EXAMPLE 1-1 | 99.5 | 1.5 |
| EXAMPLE 1-2 | 97.2 | 2.0 |
| EXAMPLE 1-3 | 100.2 | 3.1 |
| EXAMPLE 1-4 | 99.6 | 3.8 |
| EXAMPLE 1-5 | 98.5 | 3.6 |
| EXAMPLE 1-6 | 96.0 | 1.6 |
| EXAMPLE 1-7 | 103.1 | 9.4 |
| EXAMPLE 1-8 | 91.4 | 4.5 |
| EXAMPLE 1-9 | 106.1 | 5.4 |
| EXAMPLE 1-10 | 99.2 | 3.7 |
| EXAMPLE 1-11 | 102.7 | 6.5 |
| EXAMPLE 1-12 | 102.8 | 6.6 |
| EXAMPLE 1-13 | 96.1 | 4.4 |
| EXAMPLE 1-14 | 92.9 | 3.5 |
| EXAMPLE 1-15 | 99.7 | 7.3 |
| EXAMPLE 1-16 | 95.5 | 6.9 |

From Table 4, it is found that the first reagent containing the phosphonium salt compound of the present invention may maintain the reactivity of the enzymes for measuring cholesterol with the HDL fraction, while inhibiting the reactivity with the VLDL fraction, in any of Example 1-1 to Examples 1-16 indicated in Table 3. Furthermore, the degree of inhibition of the reactivity is equal to or greater than that in a case of Comparative Example 1-2 where magnesium ions are used. Because the first reagent, containing the phosphonium salt compound of the present invention, does not produce a hardly water-soluble substance even when being mixed with the alkaline cleaning liquid for the automatic analyzer, the first reagent may be said to be an extremely useful means, in relation to a case of Comparative Example 1-2 where the first reagent is used. In addition, in comparison with Comparative Example 1-3 to Comparative Example 1-5, it is found that, as to the inhibition of the reactivity with the VLDL fraction, the first reagent containing the phosphonium salt compound of the present invention is excellent.

Such an effect is also recognized when any of trimethylhexadecylphosphonium bromide, triethyldodecylphosphonium bromide, triethylhexadecylphosphonium chloride, tributyl-n-octyl phosphonium chloride, tributyldodecylphosphonium bromide, tributylhexadecylphosphonium bromide and trihexyltetradecylphosphonium bromide is used. In addition, such an effect does not depend on the kind of polyanion to be used in combination, but such a similar effect is exhibited also when either sodium phosphotungstate or dextran sulfate is used (comparison of Example 1-2 with Example 1-3, 1-4 or 1-5, or comparison of Example 1-10 with Example 1-11 or 1-12). Furthermore, when dextran sulfate is used, such a desired effect may be obtained, regardless of the molecular weight (comparison of Examples 1-3 to 1-5 with each other) . In addition, such an effect is not varied, regardless of the presence or absence of the BSA (comparison of Example 1-9 with Example 1-10).

### (Example 2) Correlation between the measured value of HDL cholesterol in a serum specimen with the first reagent to which the phosphonium salt compound of the present invention is added and the measured value of HDL cholesterol by a simple DCM

With the use of sodium phosphotungstate as a polyanion, first reagents (reagents A, B, C, D, E, F, G, H and I) containing the phosphonium salt compound of the present invention were prepared as follows.

**First reagent (reagent A)**

| | |
|---|---|
| MES-NaOH buffer solution (pH 6.5) | 100 mmol/L |
| 4-aminoantipyrine | 0.7 mmol/L |
| Sodium dextran sulfate (molecular weight of 4,000) | 0.05% by weight |
| Trimethylhexadecylphosphonium bromide | 0.1 mmol/L |
| BSA | 1.0% by weight |

**First reagent (reagent B)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Triethyldodecylphosphonium bromide | 0.2 mmol/L |
| BSA | 1.0% by weight |

**First reagent (reagent C)**

| | |
|---|---|
| MES-NaOH buffer solution (pH 6.5) | 100 mmol/L |
| 4-aminoantipyrine | 0.7 mmol/L |
| Sodium dextran sulfate (molecular weight of 4,000) | 0.05% by weight |
| Triethyldodecylphosphonium bromide | 0.2 mmol/L |
| BSA | 1.0% by weight |

**First reagent (reagent D)**

| | |
|---|---|
| MES-NaOH buffer solution (pH 6.5) | 100 mmol/L |
| 4-aminoantipyrine | 0.7 mmol/L |
| Sodium dextran sulfate (molecular weight of 4,000) | 0.05% by weight |
| Triethylhexadecylphosphonium chloride | 0.1 mmol/L |
| BSA | 1.0% by weight |

**First reagent (reagent E)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Tributyl-n-octyl phosphonium chloride | 0.8 mmol/L |
| BSA | 1.0% by weight |

**First reagent (reagent F)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Tributyldodecylphosphonium bromide | 0.1 mmol/L |
| BSA | 1.0% by weight |

**First reagent (reagent G)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Tributylhexadecylphosphonium bromide | 0.05 mmol/L |
| BSA | 1.0% by weight |

**First reagent (reagent H)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Tributylhexadecylphosphonium bromide | 0.045 mmol/L |
| BLAUNON S-207 (Patent Literature 4) | 0.045 mmol/L |
| BSA | 1.0% by weight |

**First reagent (reagent I)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Tributylhexadecylphosphonium bromide | 0.045 mmol/L |
| TERTIARY NISSAN AMINE BB (Patent Literature 6) | 0.045 mmol/L |
| BSA | 1.0% by weight |

With the reagent for measuring HDL cholesterol consisting of the first reagent of each of reagent A to reagent I and the basic second reagent described in Comparative Example 1, the concentration of HDL cholesterol in each of 90 human serum specimens was measured. The true value of the concentration of HDL cholesterol in the human serum was measured with a DCM (Non Patent Literature 3). The measured value in accordance with the DCM was indexed as X and the value of each Example as Y, and comparison was conducted of the coefficient of correlation and the equation of regression. The results are shown in Table 5. The distributions of the measured values of the lipid items of the 90 human serum specimens used in the Examples had total cholesterol (T-CHO) of 49 to 289 mg/dL, HDL cholesterol (HDL-C) of 19 to 112 mg/dL, LDL cholesterol (LDL-C) of 24 to 196 mg/dL, free cholesterol (F-CHO) of 12 to 84 mg/dL, and triglycerides (TGs) of 20 to 351 mg/dL, from which it may be said that the specimen group is generally composed of normal specimens although part of the specimens exhibits measured value out of the reference standard value. T-CHO was measured with Cholestest (registered trademark) CHO, HDL-C was done with Cholestest (registered trademark) N HDL, LDL-C was done with Cholestest (registered trademark) LDL, F-CHO was done with Pureauto (registered trademark) S F-CHO-N, and TGs were done with Cholestest (registered trademark) TG (which were all from SEKISUI MEDICAL Co., Ltd.). In addition, the specimen having TGs of 200 mg/dL or more was diluted in twice a volume with a saline, followed by subjecting to the DCM.

The specific procedure of the above mentioned DCM is as follows. For the DCM fractionation reagent, the following reagents were used: sodium dextran sulfate (Dextralip50, Sigma), magnesium chloride hexahydrate (Kishida Chemical Co., Ltd.), and sodium azide (Kishida Chemical Co., Ltd.). The measuring procedure is described as follows.

### (1) Preparation of the fraction solution

**Reagent a**

| | |
|---|---|
| Sodium dextran sulfate | 20 g/L |
| Sodium azide | 0.5 g/L, and |

**Reagent b**

| | |
|---|---|
| Magnesium chloride | 0.7 mol/L |
| Sodium azide | 0.5 g/L |

were prepared, and both of them were mixed in equal amounts.
(2) A serum sample and the fraction solution prepared in the above (1) were mixed at a volume ratio of 10 : 1, followed by mixing on a vortex mixer.
(3) The mixture was allowed to stand for 10 to 30 minutes at room temperature, and then centrifuged at 1,500 G at 4°C for 30 minutes.
(4) The supernatant was collected, and the content of the total cholesterol was measured with Cholestest (registered trademark) CHO (SEKISUI MEDICAL Co., Ltd.) . Because the serum sample was diluted in 1.1 times a volume with the fraction solution, the measured value obtained was multiplied by 1.1, to give HDL cholesterol value in the serum sample.

**[Table 5]**

| FIRST REAGENT | INCLINATION OF EQUATION OF REGRESSION | INTERCEPT OF EQUATION OF REGRESSION | COEFFICIENT OF CORRELATION R² |
|---|---|---|---|
| REAGENT A | 0.9720 | 2.6 | 0.9964 |
| REAGENT B | 0.9966 | 0.4 | 0.9961 |
| REAGENT C | 0.9622 | 3.2 | 0.9960 |
| REAGENT D | 0.9616 | 3.3 | 0.9962 |
| REAGENT E | 1.0277 | 4.0 | 0.9960 |
| REAGENT F | 0.9839 | 2.1 | 0.9942 |
| REAGENT G | 0.9907 | 6.7 | 0.9906 |
| REAGENT H | 1.0395 | 1.8 | 0.9745 |
| REAGENT I | 0.9734 | 2.8 | 0.9867 |

### (Comparative Example 2) Correlation between the measured value in accordance with the first reagent in a conventional method and the measured value in accordance with the DCM

As first reagents in accordance with conventional methods, the following first reagents (reagents J, K, L, M and N) were prepared.

**First reagent (reagent J)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |

**First reagent (reagent K)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Magnesium chloride | 8 mmol/L |

**First reagent (reagent L)**

| | |
|---|---|
| MES-NaOH buffer solution (pH 6.5) | 100 mmol/L |
| 4-aminoantipyrine | 0.7 mmol/L |
| Sodium dextran sulfate (molecular weight of 500,000) | 0.1% by weight |
| BLAUNON L-205 | 0.007% by weight |
| BSA | 0.2% by weight |

**First reagent (reagent M)**

| | |
|---|---|
| MES-NaOH buffer solution (pH 6.5) | 100 mmol/L |
| 4-aminoantipyrine | 0.7 mmol/L |
| Sodium dextran sulfate (molecular weight of 500,000) | 0.1% by weight |
| Cetyltrimethylammonium bromide | 0.002% by weight |
| BSA | 1.0% by weight |

**First reagent (reagent N)**

| | |
|---|---|
| MES-NaOH buffer solution (pH 6.5) | 100 mmol/L |
| 4-aminoantipyrine | 0.7 mmol/L |
| Sodium phosphotungstate | 0.04% by weight |
| Cetyltrimethylammonium bromide | 0.002% by weight |
| BSA | 1.0% by weight |

With the first reagent of each of reagent J to reagent N and the reagent for measuring HDL cholesterol consisting of the basic second reagent described in Comparative Example 1, the concentration of HDL cholesterol in each of 90 human serum specimens which were the same as Example 2 was measured. The measured value in accordance with the DCM was indexed as X and the value of each Example as Y, comparison was conducted of the coefficient of correlation and the equation of regression. The results are shown in Table 6.

**[Table 6]**

| FIRST REAGENT | INCLINATION OF EQUATION OF REGRESSION | INTERCEPT OF EQUATION OF REGRESSION | COEFFICIENT OF CORRELATION R² |
|---|---|---|---|
| REAGENT J | 1.0640 | 5.7 | 0.9581 |
| REAGENT K | 1.0067 | 3.3 | 0.9952 |
| REAGENT L | 0.9537 | 12.0 | 0.9811 |
| REAGENT M | 0.9898 | 5.3 | 0.9913 |
| REAGENT N | 0.9883 | -0.8 | 0.9921 |

From Example 2, in the measurement with the first reagent containing the phosphonium salt of the present invention and the polyanion, it has been found that a good correlation with the DCM was recognized. Also, for any of the first reagents of reagent A to reagent I, it has been found that the correlation is obviously significant in comparison with reagent J containing no phosphonium salt, and the correlation with the DCM in a case where the specimen group that was almost normal was measured was equal to or higher even in comparison with a case of a method with bivalent metal described in Patent Literature 3 (reagent K), a method with polyoxyethylene lauryl amine described in Patent Literature 4 (reagent L) or a method with cetyltrimethylammonium bromide described in Patent Literature 6 (reagent M and reagent N).

### (Example 3) Measurement of abnormal specimens

With the first reagent of each of reagent E to reagent I and the reagent for measuring HDL cholesterol consisting of the second reagent described in Comparative Example 1, the concentration of HDL cholesterol in each of human serum specimens indicated in Table 7 was measured. The concentrations of the human serum specimens indicated in Table 7 were measured with the reagents for measuring lipid described in Example 2. The HDL cholesterol value was calculated as follows. First of all, onto 1.0 mL of serum, a salt solution having a density of 1.006 was layered, followed by centrifugationat 26, 000 G for 30 minutes. Then, the same volume as the salt solution layered was removed from the upper layer, and the floated chylomicrons layer was removed. To the sample obtained in this way, the DCM was applied to carry out measurement. The measured value in accordance with the DCM and the measured value when each of the reagents was used are shown in Table 8.

**[Table 7]**

| | T-CHO | LDL-C | F-CHO | TG |
|---|---|---|---|---|
| SPECIMEN 1 | 110 | 18 | 72 | 1243 |
| SPECIMEN 2 | 269 | 93 | 101 | 1093 |
| SPECIMEN 3 | 151 | 50 | 53 | 992 |
| SPECIMEN 4 | 158 | 52 | 51 | 646 |
| SPECIMEN 5 | 234 | 61 | 93 | 749 |

| | | | | |
|---|---|---|---|---|
| (unit: mg/dL) | | | | |

**[Table 8]**

| | | FIRST REAGENT | | | | |
|---|---|---|---|---|---|---|
| | DCM METHOD | REAGENT E | REAGENT F | REAGENT G | REAGENT H | REAGENT I |
| SPECIMEN 1 | 8.8 | 4.8 | 4.3 | 3.8 | 4.6 | 3.3 |
| SPECIMEN 2 | 46.2 | 43.5 | 42.6 | 41.0 | 44.8 | 49.6 |
| SPECIMEN 3 | 15.4 | 16.1 | 15.7 | 16.6 | 14.3 | 15.6 |
| SPECIMEN 4 | 22.5 | 29.8 | 28.5 | 26.1 | 24.7 | 26.2 |
| SPECIMEN 5 | 19.0 | 22.0 | 22.2 | 23.3 | 17.5 | 22.0 |

| | | | | | | |
|---|---|---|---|---|---|---|
| (unit: mg/dL) | | | | | | |

### (Comparative Example 3)

With the first reagent of each of reagent J to reagent N and the reagent for measuring HDL cholesterol consisting of the basic second reagent described in Comparative Example 1, the concentration of HDL cholesterol in each of human serum specimens shown in Table 7 was measured. The measured values in accordance with the DCM and the measured values when each of the reagents was used are shown in Table 9.

**[Table 9]**

| | | FIRST REAGENT | | | | |
|---|---|---|---|---|---|---|
| | DCM METHOD | REAGENT J | REAGENT K | REAGENT L | REAGENT M | REAGENT N |
| SPECIMEN 1 | 8.8 | 8.1 | -4.3 | -21.6 | -15.5 | -3.6 |
| SPECIMEN 2 | 46.2 | 167.5 | 35.0 | 15.8 | 76.2 | 15.0 |
| SPECIMEN 3 | 15.4 | -7.1 | 22.2 | 14.1 | 19.3 | 13.5 |
| SPECIMEN 4 | 22.5 | 42.5 | 29.7 | 35.0 | 34.9 | 25.0 |
| SPECIMEN 5 | 19.0 | 53.2 | 24.0 | 31.0 | 32.1 | 19.2 |

| | | | | | | |
|---|---|---|---|---|---|---|
| (unit: mg/dL) | | | | | | |

When the IV-type hyperlipidemia-like specimens indicated in Table 7, in each of which triglycerides were included at an abnormally high value, whereas total cholesterol and LDL cholesterol were included at almost normal value, were measured with each of reagent J to reagent N described in Comparative Example, there arose a large difference from the DCM value, as shown in Table 9. In contrast, also when the specimens shown in Table 7 were measured with each of reagent E to reagent I containing the phosphonium salt of the present invention and the polyanion, values matching well with the DCM values were indicated. This is because it is believed that for the specimens whose total cholesterol and LDL cholesterol had an almost normal value, and which contain a lot of chylomicrons or VLDL, the conventional methods did not allow the chylomicrons or VLDL to be treated appropriately, whereas use of the phosphonium salt of the present invention in combination with the polyanion allowed the chylomicrons or VLDL to be treated more surely, so that the accuracy of measurement was improved.

### (Example 4) Combination with a surfactant

Examination was conducted on kinds of surfactants.

With samples of HDL fractions or VLDL fractions prepared through ultracentrifugation, and which were those diluted with a saline such that the concentration of cholesterol in each of the fractions became 50 mg/dL (hereinafter, the samples are also referred to as HDL fraction samples or VLDL fraction samples), the influence in a case where the kind of the surfactant to be added into the second reagent was varied was investigated. As to the second reagent, in the basic second reagent described in Comparative Example 1, instead of EMULGEN B-66 being added, reagents to which surfactants indicated in Table 10 were added were used. As to the first reagent, reagent J of Comparative Example 2 was used as a conventional method, and reagent F of Example 2 containing tributyldodecylphosphonium bromide was used as a reference reagent to which the phosphonium salt compound of the present invention was added.

**Second reagent**

| | |
|---|---|
| MES-NaOH buffer solution (pH 6.5) | 100 mmol/L |
| Cholesterol esterase | 1 U/mL |
| Cholesterol oxidase | 1 U/mL |
| Peroxidase | 5 U/mL |
| TOOS | 0.1 mmol/L |
| A variety of surfactants | At a concentration shown in Table 10 |

**[Table 10]**

| SECOND REAGENT | SURFACTANT (PRODUCT NAME) | CONCENTRATION (% BY WEIGHT) |
|---|---|---|
| EXAMPLE 3-1 | PEGNOL 005 | 1.0 |
| EXAMPLE 3-2 | EMULGEN A-60 | 0.1 |
| EXAMPLE 3-3 | NEWCOL 610 | 1.0 |
| EXAMPLE 3-4 | NEWCOL 2600FB | 1.0 |
| EXAMPLE 3-5 | ENAGICOL L-30AN | 0.5 |
| EXAMPLE 3-6 | SODIUM CHOLATE | 0.5 |
| EXAMPLE 3-7 | AMPHITOL 24B | 0.7 |
| EXAMPLE 3-8 | ENAGICOL C-40H | 0.6 |

The absorbance of the HDL fraction sample measured with reagent J as the first reagent was indexed as 100, and the relative absorbance (%) of the HDL fraction sample measured with reagent F as the first reagent to which the phosphonium salt compound of the present invention was added was calculated in the test where each of the second reagents of Example 3-1 to Example 3-8 was used. Furthermore, in each measurement, the relative absorbance (%) of the VLDL fraction sample with respect to the absorbance of the HDL fraction sample was calculated. A condition where the relative absorbance of the HDL fraction sample is large and the relative absorbance of the VLDL fraction sample/HDL fraction sample is small is a condition required to achieve an object, that is a condition where the reactivity of the enzymes for measuring cholesterol with HDL is maintained, while the reactivity of the enzymes for measuring cholesterol with VLDL is lowered. The results are shown in Table 11.

**[Table 11]**

| NAME OF SECOND REAGENT | RELATIVE ABSORBANCE (%) OF HDL FRACTION SAMPLE FOR FIRST REAGENT▪ REAGENT F/FIRST REAGENT▪ REAGENT J | RELATIVE ABSORBANCE (%) OF VLDL FRACTION SAMPLE/HDL FRACTION SAMPLE | |
|---|---|---|---|
| | | FIRST REAGENT▪ REAGENT J | FIRST REAGENT▪ REAGENT F |
| EXAMPLE 3-1 | 99.3 | 20.1 | 3.4 |
| EXAMPLE 3-2 | 80.1 | 94.5 | 23.2 |
| EXAMPLE 3-3 | 98.8 | 27.3 | 5.8 |
| EXAMPLE 3-4 | 99.9 | 25.7 | 2.0 |
| EXAMPLE 3-5 | 118.6 | 108.2 | 12.5 |
| EXAMPLE 3-6 | 117.0 | 635.5 | 3.4 |
| EXAMPLE 3-7 | 108.7 | 102.3 | 45.9 |
| EXAMPLE 3-8 | 105.0 | 171.7 | 7.1 |

Among the second reagents to each of which the same surfactant was added, comparison was conducted of the effect of adding the phosphonium salt compound of the present invention in the first reagent. In a case where the second reagent containing Pegnol 005 (Example 3-1), EMULGENA-60 (Example 3-2), Newcol 610 (Example 3-3) or Newcol 2600FB (Example 3-4) that was a non-ionic surfactant was used, in the same way as the case where EMULGEN B-66 was used (Example 2), a desired reaction was recognized in which addition of the phosphonium salt compound of the present invention together with a polyanion to the first reagent allowed the reactivity with the HDL fraction to be almost maintained, while lowering the relative absorbance of the VLDL fraction sample/the HDL fraction sample, in other words inhibited the reactivity of the enzymes for measuring cholesterol with the VLDL fraction. When ENAGICOL L-30AN (Example 3-5) or sodium cholate (Example 3-6) that was an anionic surfactant was used, with the measurement of the HDL fraction sample in which reagent J was used as the first reagent, increase of the absorbance had not been saturated within a predetermined reaction time and thus the color reaction had not been completed (data not shown). However, when reagent F of the present invention was used as the first reagent, the reactivity with the HDL fraction was improved, so that the absorbance ratio of the HDL fraction of reagent F/reagent J had a value beyond 100%. In addition, the reactivity of the enzymes for measuring cholesterol with the VLDL fraction when reagent J was used as the first reagent was equal to or greater than that with the HDL fraction in a case where ENAGICOL L-30AN was used (108.2%), whereas the reactivity with the VLDL fraction when sodium cholate was used was 6 times higher (635.5%) than that with the HDL fraction. However, when reagent F of the present invention was used as the first reagent, the reactivity of the VLDL fraction was greatly inhibited by any of the surfactants, so that the absorbance ratio of the VLDL fraction sample/the HDL fraction sample was small (12.5%, 3.4%) .

Also, when AMPHITOL 24B (Example 3-7) or ENAGICOL C-40H (Example 3-8) that was an amphoteric surfactant was used, the similar phenomenon to the case where an anionic surfactant was used was recognized. In other words, addition of the phosphonium salt compound of the present invention together with a polyanion to the first reagent allows the reactivity with the HDL fraction to be almost maintained (108.7%, 105.0%), while lowering the relative absorbance of the VLDL fraction sample/the HDL fraction sample (102% → 45.9%, 171.7% → 7.1%).

From the above, it has been demonstrated that the effect by which the reactivity of enzymes for measuring cholesterol with VLDL is inhibited in a method including treating LDL and VLDL in the coexistence of the phosphonium salt compound of the present invention and a polyanion with lipoproteins is obtained also in the coexistence of any surfactant for use in solubilizing HDL.

## Claims

1. A method for measuring high-density lipoprotein cholesterol in a measurement sample, comprising the step of reacting the measurement sample and cholesterol ester hydrolase and cholesterol oxidase, or cholesterol ester hydrolase and cholesterol dehydrogenase, in the presence of a compound represented by general formula (I): 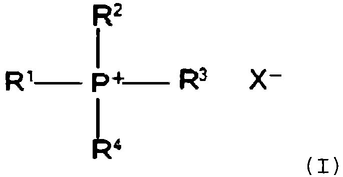
wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion,
and a polyanion.

2. The method according to claim 1, wherein in the compound represented by general formula (I), R¹ represents a linear or branched alkyl group having 8 to 16 carbon atoms, and each of R², R³ and R⁴ is a linear or branched alkyl group having 1 to 4 carbon atoms.

3. The method according to claim 1 or 2, wherein the compound represented by general formula (I) is at least one compound selected from the group consisting of a trimethyloctylphosphonium salt, a trimethyldodecylphosphonium salt, a trimethylhexadecylphosphonium salt, a triethyloctylphosphonium salt, a triethyldodecylphosphonium salt, a triethylhexadecylphosphonium salt, a tributyloctylphosphonium salt, a tributyldodecylphosphonium salt and a tributylhexadecylphosphonium salt.

4. The method according to any one of claims 1 to 3, wherein the polyanion is sodium phosphotungstate or dextran sulfate.

5. The method according to any one of claims 1 to 4, wherein the step is carried out further in the presence of albumin.

6. The method according to any one of claims 1 to 5, wherein the step is carried out further in the presence of at least one compound selected from the group consisting of a polyoxyethylene alkylamine, a polyoxyethylene alkenylamine, a tertiary amine and a quaternary ammonium salt.

7. A reagent for measuring high-density lipoprotein cholesterol in a measurement sample, comprising the following (a), (b) and (c):
(a) a compound represented by general formula (I): 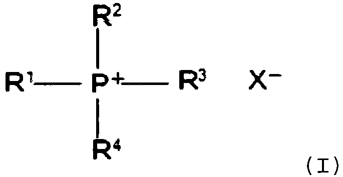 wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion,
(b) a polyanion, and
(c) cholesterol ester hydrolase and cholesterol oxidase, or cholesterol ester hydrolase and cholesterol dehydrogenase.

8. The reagent according to claim 7, wherein in the compound represented by general formula (I), R¹ represents a linear or branched alkyl group having 8 to 16 carbon atoms, and each of R², R³ and R⁴ is a linear or branched alkyl group having 1 to 4 carbon atoms.

9. The reagent according to claim 7 or 8, wherein the compound represented by general formula (I) is at least one compound selected from the group consisting of a trimethyloctylphosphonium salt, a trimethyldodecylphosphonium salt, a trimethylhexadecylphosphonium salt, a triethyloctylphosphonium salt, a triethyldodecylphosphonium salt, a triethylhexadecylphosphonium salt, a tributyloctylphosphonium salt, a tributyldodecylphosphonium salt and a tributylhexadecylphosphonium salt.

10. The reagent according to any one of claims 7 to 9, wherein the polyanion is sodium phosphotungstate or dextran sulfate.

11. The reagent according to any one of claims 7 to 10, further comprising albumin.

12. The reagent according to any one of claims 7 to 11, further comprising at least one compound selected from the group consisting of a polyoxyethylene alkylamine, a polyoxyethylene alkenylamine, a tertiary amine and a quaternary ammonium salt.

13. The reagent according to any one of claims 7 to 12, comprising
a first reagent set containing (a) the compound represented by general formula (I) and (b) the polyanion, and
a second reagent set containing (c) cholesterol ester hydrolase and cholesterol oxidase, or cholesterol ester hydrolase and cholesterol dehydrogenase.

14. A kit for use in high-density lipoprotein cholesterol measurement, comprising
(a) a compound represented by general formula (I): 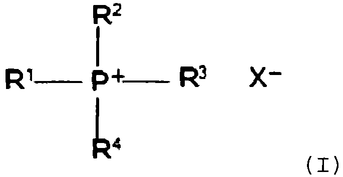 wherein R¹ represents a linear or branched alkyl group having 8 to 22 carbon atoms or a linear or branched alkenyl group having 8 to 22 carbon atoms, R², R³ and R⁴ are the same as or different from each other and represent a linear or branched alkyl group having 1 to 6 carbon atoms or a linear or branched alkenyl group having 2 to 6 carbon atoms, and X⁻ represents an anion,
(b) a polyanion, and
(c) cholesterol ester hydrolase and cholesterol oxidase, or cholesterol ester hydrolase and cholesterol dehydrogenase.

15. The kit according to claim 14, comprising
a first reagent set containing (a) the compound represented by general formula (I) and (b) the polyanion, and
a second reagent set containing (c) cholesterol ester ydrolase and cholesterol oxidase, or cholesterol ester hydrolase and cholesterol dehydrogenase.

## Patentansprüche

1. Verfahren zur Messung von High Density Lipoprotein-Cholesterin in einer Messprobe, umfassend einen Schritt des Umsetzens der Messprobe und Cholesterinester-Hydrolase und Cholesterin-Oxidase oder Cholesterinester-Hydrolase und Cholesterin-Dehydrogenase in Anwesenheit einer Verbindung der allgemeinen Formel (I): 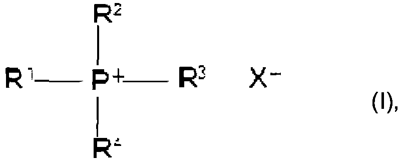
wobei R¹ für eine lineare oder verzweigte Alkylgruppe mit 8 bis 22 Kohlenstoffatomen oder eine lineare oder verzweigte Alkenylgruppe mit 8 bis 22 Kohlenstoffatomen steht,
R², R³ und R⁴ gleich oder voneinander verschieden sind und für eine lineare oder verzweigte Alkylgruppe mit 1 bis 6 Kohlenstoffatomen oder eine lineare oder verzweigte Alkenylgruppe mit 2 bis 6 Kohlenstoffatomen stehen und X⁻ für ein Anion steht, und ein Polyanion.

2. Verfahren nach Anspruch 1, wobei in der Verbindung der allgemeinen Formel (I) R¹ für eine lineare oder verzweigte Alkylgruppe mit 8 bis 16 Kohlenstoffatomen steht und jeder von R², R³ und R⁴ für eine lineare oder verzweigte Alkylgruppe mit 1 bis 4 Kohlenstoffatomen steht.

3. Verfahren nach Anspruch 1 oder 2, wobei die Verbindung der allgemeinen Formel (I) wenigstens eine Verbindung ist, ausgewählt aus der Gruppe, bestehend aus einem Trimethyloctylphosphoniumsalz, einem Trimethyldodecylphosphoniumsalz, einem Trimethylhexadecylphosphoniumsalz, einem Triethyloctylphosphoniumsalz, einem Triethyldodecylphosphoniumsalz, einem Triethylhexadecylphosphoniumsalz, einem Tributyloctylphosphoniumsalz, einem Tributyldodecylphosphoniumsalz und einem Tributylhexadecylphosphoniumsalz.

4. Verfahren nach einem der Ansprüche 1 bis 3, wobei das Polyanion Natriumphosphowolframat oder Dextransulfat ist.

5. Verfahren nach einem der Ansprüche 1 bis 4, wobei der Schritt ferner in Anwesenheit von Albumin durchgeführt wird.

6. Verfahren nach einem der Ansprüche 1 bis 5, wobei der Schritt ferner in Anwesenheit wenigstens einer Verbindung durchgeführt wird, ausgewählt aus der Gruppe, bestehend aus einem Polyoxyethylenalkylamin, einem Polyoxyethylenalkenylamin, einem tertiären Amin und einem quartären Ammoniumsalz.

7. Reagenz zur Messung von High Density Lipoprotein-Cholesterin in einer Messprobe, umfassend das folgende (a), (b) und (c):
(a) eine Verbindung der allgemeinen Formel (I): 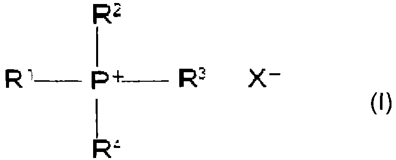
wobei R¹ für eine lineare oder verzweigte Alkylgruppe mit 8 bis 22 Kohlenstoffatomen oder eine lineare oder verzweigte Alkenylgruppe mit 8 bis 22 Kohlenstoffatomen steht,
R², R³ und R⁴ gleich oder voneinander verschieden sind und für eine lineare oder verzweigte Alkylgruppe mit 1 bis 6 Kohlenstoffatomen oder eine lineare oder verzweigte Alkenylgruppe mit 2 bis 6 Kohlenstoffatomen stehen und X⁻ für ein Anion steht,
(b) ein Polyanion und
(c) Cholesterinester-Hydrolase und Cholesterin-Oxidase oder Cholesterinester-Hydrolase und Cholesterin-Dehydrogenase.

8. Reagenz nach Anspruch 7, wobei in der Verbindung der allgemeinen Formel (I) R¹ für eine lineare oder verzweigte Alkylgruppe mit 8 bis 16 Kohlenstoffatomen steht und jeder von R², R³ und R⁴ für eine lineare oder verzweigte Alkylgruppe mit 1 bis 4 Kohlenstoffatomen steht.

9. Reagenz nach Ansprüche 7 oder 8, wobei die Verbindung der allgemeinen Formel (I) wenigstens eine Verbindung ist, ausgewählt aus der Gruppe, bestehend aus einem Trimethyloctylphosphoniumsalz, einem Trimethyldodecylphosphoniumsalz, einem Trimethylhexadecylphosphoniumsalz, einem Triethyloctylphosphoniumsalz, einem Triethyldodecylphosphoniumsalz, einem Triethylhexadecylphosphoniumsalz, einem Tributyloctylphosphoniumsalz, einem Tributyldodecylphosphoniumsalz und einem Tributylhexadecylphosphoniumsalz.

10. Reagenz nach einem der Ansprüche 7 bis 9, wobei das Polyanion Natriumphosphowolframat oder Dextransulfat ist.

11. Reagenz nach einem der Ansprüche 7 bis 10, ferner Albumin umfassend.

12. Reagenz nach einem der Ansprüche 7 bis 11, ferner wenigstens eine Verbindung umfassend, ausgewählt aus der Gruppe, bestehend aus einem Polyoxyethylenalkylamin, einem Polyoxyethylenalkenylamin, einem tertiären Amin und einem quartären Ammoniumsalz.

13. Reagenz nach einem der Ansprüche 7 bis 12, umfassend einen ersten Reagenziensatz, der (a) eine Verbindung der allgemeinen Formel (I) und (b) ein Polyanion umfasst, und einen zweiten Reagenziensatz, der (c) Cholesterinester-Hydrolase und Cholesterin-Oxidase oder Cholesterinester-Hydrolase und Cholesterin-Dehydrogenase umfasst.

14. Kit zur Verwendung bei der Messung von High Density Lipoprotein-Cholesterin, umfassend
(a) eine Verbindung der allgemeinen Formel (I): 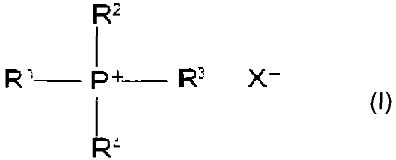
wobei R¹ für eine lineare oder verzweigte Alkylgruppe mit 8 bis 22 Kohlenstoffatomen oder eine lineare oder verzweigte Alkenylgruppe mit 8 bis 22 Kohlenstoffatomen steht,
R², R³ und R⁴ gleich oder voneinander verschieden sind und für eine lineare oder verzweigte Alkylgruppe mit 1 bis 6 Kohlenstoffatomen oder eine lineare oder verzweigte Alkenylgruppe mit 2 bis 6 Kohlenstoffatomen stehen und X⁻ für ein Anion steht,
(b) ein Polyanion und
(c) Cholesterinester-Hydrolase und Cholesterin-Oxidase oder Cholesterinester-Hydrolase und Cholesterin-Dehydrogenase.

15. Kit nach Anspruch 14, umfassend einen ersten Reagenziensatz, der (a) eine Verbindung der allgemeinen Formel (I) und (b) ein Polyanion umfasst, und einen zweiten Reagenziensatz, der (c) Cholesterinester-Hydrolase und Cholesterin-Oxidase oder Cholesterinester-Hydrolase und Cholesterin-Dehydrogenase umfasst.

## Revendications

1. Procédé pour mesurer le cholestérol de lipoprotéine haute densité dans un échantillon de mesure, comprenant l'étape consistant à faire réagir l'échantillon de mesure et de l'ester de cholestérol hydrolase et de la cholestérol oxydase, ou de l'ester de cholestérol hydrolase et de la cholestérol déshydrogénase, en présence d'un composé représenté par la formule générale (I) : 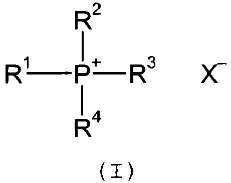
dans laquelle R¹ représente un groupe alkyle linéaire ou ramifié possédant 8 à 22 atomes de carbone ou un groupe alcényle linéaire ou ramifié possédant 8 à 22 atomes de carbone, R², R³ et R⁴ sont identiques ou différents et représentent un groupe alkyle linéaire ou ramifié possédant 1 à 6 atomes de carbone ou un groupe alcényle linéaire ou ramifié possédant 2 à 6 atomes de carbone, et X⁻ représente un anion,
et un polyanion.

2. Procédé selon la revendication 1, dans lequel, dans le composé représenté par la formule générale (I), R¹ représente un groupe alkyle linéaire ou ramifié possédant 8 à 16 atomes de carbone, et chacun de R², R³ et R⁴ est un groupe alkyle linéaire ou ramifié possédant 1 à 4 atomes de carbone.

3. Procédé selon la revendication 1 ou 2, dans lequel le composé représenté par la formule générale (I) est au moins un composé choisi dans l'ensemble constitué par un sel de triméthyloctylphosphonium, un sel de triméthyldodécylphosphonium, un sel de triméthylhexadécylphosphonium, un sel de triéthyloctylphosphonium, un sel de triéthyldodécylphosphonium, un sel de triéthylhexadécylphosphonium, un sel de tributyloctylphosphonium, un sel de tributyldodécylphosphonium et un sel de tributylhexadécylphosphonium.

4. Procédé selon l'une quelconque des revendications 1 à 3, dans lequel le polyanion est le phosphotungstate de sodium ou le sulfate de dextrane.

5. Procédé selon l'une quelconque des revendications 1 à 4, dans lequel l'étape est effectuée en outre en présence d'albumine.

6. Procédé selon l'une quelconque des revendications 1 à 5, dans lequel l'étape est effectuée en outre en présence d'au moins un composé choisi dans l'ensemble constitué par une alkylamine polyoxyéthylénée, une alcénylamine polyoxyéthylénée, une amine tertiaire et un sel d'ammonium quaternaire.

7. Réactif pour mesurer le cholestérol de lipoprotéine haute densité dans un échantillon de mesure, comprenant les points (a), (b) et (c) suivants :
(a) un composé représenté par la formule générale (I) : 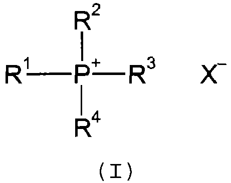 dans laquelle R¹ représente un groupe alkyle linéaire ou ramifié possédant 8 à 22 atomes de carbone ou un groupe alcényle linéaire ou ramifié possédant 8 à 22 atomes de carbone, R², R³ et R⁴ sont identiques ou différents et représentent un groupe alkyle linéaire ou ramifié possédant 1 à 6 atomes de carbone ou un groupe alcényle linéaire ou ramifié possédant 2 à 6 atomes de carbone, et X⁻ représente un anion,
(b) un polyanion, et
(c) de l'ester de cholestérol hydrolase et de la cholestérol oxydase, ou de l'ester de cholestérol hydrolase et de la cholestérol déshydrogénase.

8. Réactif selon la revendication 7, dans lequel, dans le composé représenté par la formule générale (I), R¹ représente un groupe alkyle linéaire ou ramifié possédant 8 à 16 atomes de carbone, et chacun de R², R³ et R⁴ est un groupe alkyle linéaire ou ramifié possédant 1 à 4 atomes de carbone.

9. Réactif selon la revendication 7 ou 8, dans lequel le composé représenté par la formule générale (I) est au moins un composé choisi dans l'ensemble constitué par un sel de triméthyloctylphosphonium, un sel de triméthyldodécylphosphonium, un sel de triméthylhexadécylphosphonium, un sel de triéthyloctylphosphonium, un sel de triéthyldodécylphosphonium, un sel de triéthylhexadécylphosphonium, un sel de tributyloctylphosphonium, un sel de tributyldodécylphosphonium et un sel de tributylhexadécylphosphonium.

10. Réactif selon l'une quelconque des revendications 7 à 9, dans lequel le polyanion est le phosphotungstate de sodium ou le sulfate de dextrane.

11. Réactif selon l'une quelconque des revendications 7 à 10, comprenant en outre de l'albumine.

12. Réactif selon l'une quelconque des revendications 7 à 11, comprenant en outre au moins un composé choisi dans l'ensemble constitué par une alkylamine polyoxyéthylénée, une alcénylamine polyoxyéthylénée, une amine tertiaire et un sel d'ammonium quaternaire.

13. Réactif selon l'une quelconque des revendications 7 à 12, comprenant
un premier jeu de réactifs contenant (a) le composé représenté par la formule générale (I) et (b) le polyanion, et
un deuxième jeu de réactifs contenant (c) de l'ester de cholestérol hydrolase et de la cholestérol oxydase, ou de l'ester de cholestérol hydrolase et de la cholestérol déshydrogénase.

14. Kit pour une utilisation dans la mesure de cholestérol de lipoprotéine haute densité, comprenant
(a) un composé représenté par la formule générale (I) : 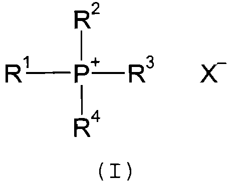 dans laquelle R¹ représente un groupe alkyle linéaire ou ramifié possédant 8 à 22 atomes de carbone ou un groupe alcényle linéaire ou ramifié possédant 8 à 22 atomes de carbone, R², R³ et R⁴ sont identiques ou différents et représentent un groupe alkyle linéaire ou ramifié possédant 1 à 6 atomes de carbone ou un groupe alcényle linéaire ou ramifié possédant 2 à 6 atomes de carbone, et X⁻ représente un anion,
(b) un polyanion, et
(c) de l'ester de cholestérol hydrolase et de la cholestérol oxydase, ou de l'ester de cholestérol hydrolase et de la cholestérol déshydrogénase.

15. Kit selon la revendication 14, comprenant un premier jeu de réactifs contenant (a) le composé représenté par la formule générale (I) et (b) le polyanion, et
un deuxième jeu de réactifs contenant (c) de l'ester de cholestérol hydrolase et de la cholestérol oxydase, ou de l'ester de cholestérol hydrolase et de la cholestérol déshydrogénase.
